# EUROPEAN PATENT APPLICATION

(11) **EP 4 137 079 A1**
(43) Date of publication of application: **22.02.2023**
(21) Application number: 22190346.1
(22) Date of filing: 15.08.2022
(51) Int. Cl.: A61B 18/14, A61B 18/00, A61B 5/06, A61B 5/113, A61B 5/00

(54) **PHRENIC NERVE WARNING**

(30) Priority: 16.08.2021 US 202117403112
(71) Applicant: Biosense Webster (Israel) Ltd, Yokneam, 2066717 (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(57) **Abstract**

In one embodiment, an ablation system includes a catheter including at least one electrode, and configured to be inserted into a chamber of a heart of a living subject, an ablation power generator configured to apply an electrical signal to the at least one electrode to ablate tissue of the chamber, at least one body surface patch configured to be applied to a body surface of the living subject, and provide at least one position signal, and a processor configured to compute an index of a measurement of diaphragm movement responsively to the at least one position signal, and perform an action responsively to the computed index.

## Description

### FIELD OF THE INVENTION

The present invention relates to medical systems, and in particular, but not exclusively, to cardiac ablation.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/0065455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied through the tip electrode(s) of the ablating catheter, and current flows through the media that surrounds it, i.e., blood and tissue, between the tip electrode(s) and an indifferent electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

Irreversible electroporation (IRE) applies short electrical pulses that generate high enough electrical fields (typically greater than 450 Volts per centimeter) to irreversibly damage the cells. Non-thermal IRE may be used in treating different types of tumors and other unwanted tissue without causing thermal damage to surrounding tissue. Small electrodes are placed in proximity to target tissue to apply short electrical pulses. The pulses increase the resting transmembrane potential, so that nanopores form in the plasma membrane. When the electricity applied to the tissue is above the electric field threshold of the target tissue, the cells become permanently permeable from the formation of nanopores. As a result, the cells are unable to repair the damage and die due to a loss of homeostasis and the cells typically die by apoptosis.

IRE may be used for cardiac ablation as an alternative to other cardiac ablation techniques, e.g., radio frequency (RF) cardiac ablation. IRE cardiac ablation is sometimes referred to as Pulse Field Ablation (PFA). As IRE is generally a low thermal technique, IRE may reduce the risk of collateral cell damage that is present with the other techniques. e.g., in RF cardiac ablation.

### SUMMARY

There is provided in accordance with still another embodiment of the present disclosure, a ablation system, including a catheter including at least one electrode, and configured to be inserted into a chamber of a heart of a living subject, an ablation power generator configured to apply an electrical signal to the at least one electrode to ablate tissue of the chamber, at least one body surface patch configured to be applied to a body surface of the living subject, and provide at least one position signal, and a processor configured to compute an index of a measurement of diaphragm movement responsively to the at least one position signal, and perform an action responsively to the computed index.

Further in accordance with an embodiment of the present disclosure the processing is configured to control the ablation power generator responsively to the computed index.

Still further in accordance with an embodiment of the present disclosure the processing is configured to output an alert to an output device responsively to the computed index.

Additionally in accordance with an embodiment of the present disclosure the processor is configured to output the alert to the output device responsively to the computed index exceeding a given threshold.

Moreover, in accordance with an embodiment of the present disclosure the processor is configured to receive a user input setting the given threshold.

Further in accordance with an embodiment of the present disclosure the alert is selected from a group consisting of a current value of the index, an alert message, a tactile alert, and an audio alert.

Still further in accordance with an embodiment of the present disclosure the processor is configured to compute indices of the measurement of diaphragm movement over time responsively to at least one of the position signals, and output the alert to the output device while adjusting a strength of the alert responsively to the computed indices over time.

Additionally in accordance with an embodiment of the present disclosure the index of the measurement of the diaphragm movement is indicative of proximity of the at least one electrode to the phrenic nerve.

Moreover in accordance with an embodiment of the present disclosure, the system includes magnetic field generator coils configured to generate alternating magnetic fields in a region including the at least one body surface patch, wherein the at least one body surface patch includes at least one magnetic sensor configured to provide the at least one position signal responsively to sensing the generated alternating magnetic fields, and the processor is configured to compute the index of the measurement of the diaphragm movement responsively to the at least one position signal received from the at least magnetic sensor.

Further in accordance with an embodiment of the present disclosure the at least one body surface patch includes at least one respective electrode configured to provide the at least one position signal, and the processor is configured to compute the index of the measurement of the diaphragm movement responsively to the at least one position signal received from the at least one respective electrode.

Still further in accordance with an embodiment of the present disclosure the processor is configured to compute the index of the measurement of the diaphragm movement responsively to a measurement of velocity of the diaphragm movement.

Additionally in accordance with an embodiment of the present disclosure the processor is configured to compute the index of the measurement of the diaphragm movement responsively to a measurement of acceleration of the diaphragm movement.

There is also provided in accordance with another embodiment of the present disclosure, a ablation method, including applying an electrical signal to at least one electrode of a catheter inserted into a chamber of a heart of a living subject to ablate tissue of the chamber, providing at least one position signal by at least one body surface patch attached to a body surface of the living subject, computing an index of a measurement of diaphragm movement responsively to the at least one position signal, and performing an action responsively to the computed index.

Moreover, in accordance with an embodiment of the present disclosure, the method includes controlling an ablation power generator responsively to the computed index.

Further in accordance with an embodiment of the present disclosure, the method includes outputting an alert to an output device responsively to the computed index.

Still further in accordance with an embodiment of the present disclosure the outputting includes outputting the alert to the output device responsively to the computed index exceeding a given threshold.

Additionally in accordance with an embodiment of the present disclosure, the method includes receiving a user input setting the given threshold.

Moreover, in accordance with an embodiment of the present disclosure the alert is selected from a group consisting of a current value of the index, an alert message, a tactile alert, and an audio alert.

Further in accordance with an embodiment of the present disclosure, the method includes computing indices of the measurement of diaphragm movement over time responsively to at least one of the position signals, wherein the outputting includes outputting the alert to the output device while adjusting a strength of the alert responsively to the computed indices over time.

Still further in accordance with an embodiment of the present disclosure the index of the measurement of the diaphragm movement is indicative of proximity of the at least one electrode to the phrenic nerve.

Additionally in accordance with an embodiment of the present disclosure, the method includes generating alternating magnetic fields in a region including the at least one body surface patch, wherein the providing includes at least one magnetic sensors of the at least one body surface patch providing the at least one position signal responsively to sensing the generated alternating magnetic fields, and the computing includes computing the index of the measurement of the diaphragm movement responsively to the at least one position signal received from the at least magnetic sensor.

Moreover, in accordance with an embodiment of the present disclosure the providing includes at least one respective electrode of the at least one body surface patch providing the at least one position signal, and the computing includes computing the index of the measurement of the diaphragm movement responsively to the at least one position signal received from the at least one respective electrode.

Further in accordance with an embodiment of the present disclosure the computing includes computing the index of the measurement of the diaphragm movement responsively to a measurement of velocity of the diaphragm movement.

Still further in accordance with an embodiment of the present disclosure the computing includes computing the index of the measurement of the diaphragm movement responsively to a measurement of acceleration of the diaphragm movement.

### BRIEF DESCRIPTION OF THE DRAWINGS

The present invention will be understood from the following detailed description, taken in conjunction with the drawings in which:
Fig. 1 is a schematic pictorial illustration of a catheter-based position tracking and ablation system in accordance with an exemplary embodiment of the present invention;
Fig. 2 is a schematic pictorial illustration of a balloon catheter used in the system of Fig. 1; and
Fig. 3 is a flowchart including a method of operation of the system of Fig. 1.

### DESCRIPTION OF EXAMPLE EMBODIMENTS

### OVERVIEW

During an ablation procedure, there is a concern that the region being ablated is too close to the phrenic nerve, and that ablation may damage the nerve. A physician may test for proximity of the ablation electrode(s) to the phrenic nerve by applying an electrical signal (e.g., a pacing signal) to the ablation electrode(s) while the physician observes if the electrical signal induces diaphragm motion. However, this relies on the surgeon observing the motion of the diaphragm. For example, sometimes, during RF ablation, for example, of the pulmonary vein (PV), the physician may drag the catheter around the PV and may get too close to the phrenic without realizing.

Therefore, embodiments of the present of the invention solve the above problem by automatically evaluating diaphragm movement by computing an index of measurement of diaphragm movement and performing an action responsively to the computed index such as controlling an ablation power generator to stop or prevent ablation, or provide an alert. The index is indicative of proximity of the catheter electrode(s) to the phrenic nerve. Therefore, the alert provides warning to the physician about proximity of the catheter electrode(s) to the phrenic nerve.

The alert may be provided responsively to the index exceeding a threshold, e.g., set by the physician. The alert may comprise any one or more of the following: a current value of the index; an alert message displayed on a display screen; a tactile alert provided to the physician e.g., via a handle of the catheter held by the physician, and/or via a foot peddle operated by the physician; an audio alert (e.g., an alarm and/or a verbal warning); and a graph of the value of the index against time.

In some embodiments, the strength and/or frequency of the alert may increase (e.g., the alarm becomes louder and/or has a higher frequency, or the tactile alert becomes stronger and/or has a higher frequency, or the display message becomes brighter, bigger, or a stronger color) according to the value of the index, so that as the catheter electrode(s) get closer to the phrenic nerve the strength and/or frequency of the alert increases.

The index may be computed according to displacement, velocity, and/or acceleration of the diaphragm movement. The diaphragm movement may be indicated based one or more position signals provided by one or more position sensors disposed in one or more respective body surface patches applied to the body surface (e.g., chest and/or back) of the patient.

In some embodiments, magnetic field generator coils generate alternating magnetic fields in a region including the body surface patch(es), which include one or more respective magnetic sensors. The magnetic sensor(s) may provide position signal(s) responsively to sensing the generated alternating magnetic fields. One or more of the position signals are then processed to compute the position(s) of the corresponding body surface patch(es). The computed position(s) may then be used to compute the index of diaphragm movement. For example, the computed positions may be summed, averaged or otherwise combined to compute the index. The positions may be relative positions, for example, relative to an origin of a magnetic coordinate system, relative to an average position of the chest or diaphragm, or relative to any suitable position. In the above computation of the index, the position of the body surface patch closest to the diaphragm on the chest may be used. In some embodiments, the positions of a sub-set of the body surface patches closest to the diaphragm may be used in computing the index. In some embodiments, the computation of the index may weight the computed positions of the (subset of) body surface patches according to the proximity of the corresponding body surface patches to the diaphragm providing a higher weight to patches closer to the diaphragm.

In some embodiments, the body surface patches include respective electrodes which provide position signals. The electrodes may detect signals provided by the catheter electrode(s), and/or from other one or ones of the body surface patches, and/or from a reference electrode placed in the heart or on the back of the patient, for example. The position signals may then be used to compute positions of one or more of the body surface patches. One or more of the computed positions may then be used to compute the index as described above with reference to the positions computed for the magnetic sensors.

In some embodiments, a distribution of current or impedance values over the body surface patch electrodes may provide an indication of diaphragm movement. Therefore, the index of diaphragm movement may be computed based on the distribution of the current or impedance values of all, or a subset of, the body surface patch electrodes.

### SYSTEM DESCRIPTION

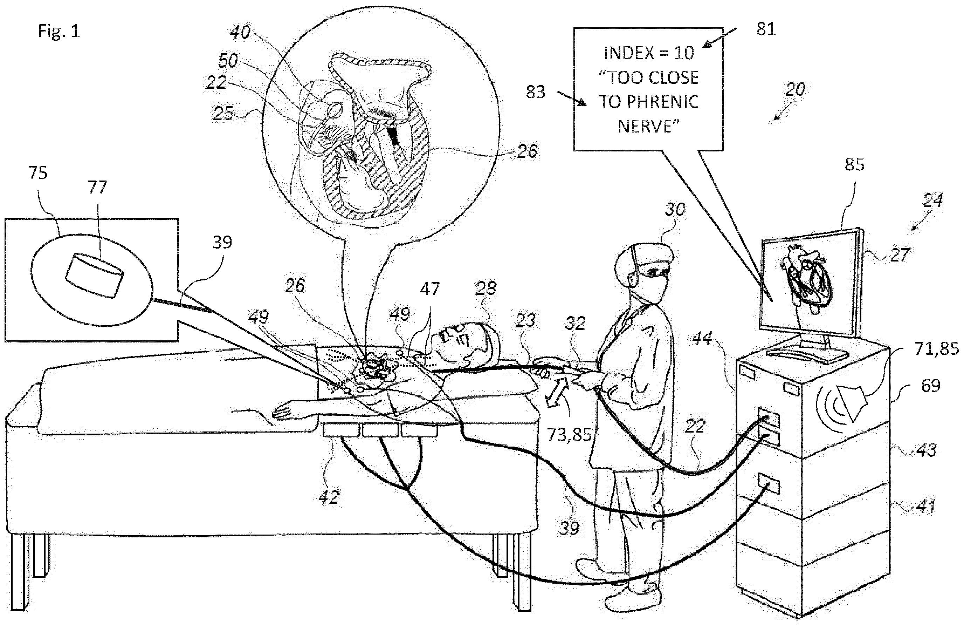

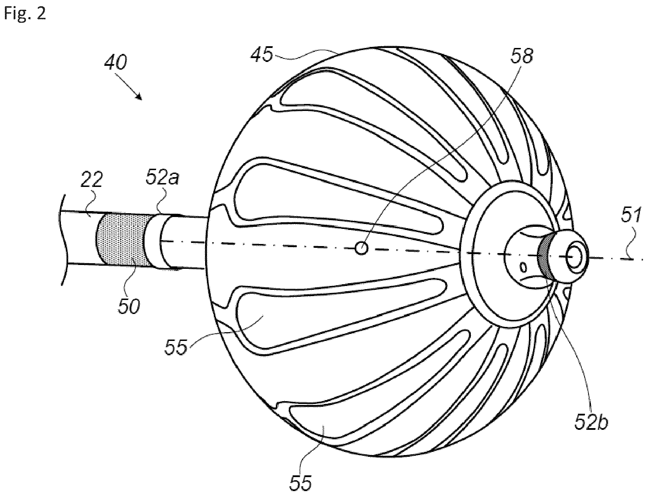

Reference is now made to Fig. 1, which is a schematic pictorial illustration of a catheter-based position tracking and ablation system 20 in accordance with an exemplary embodiment of the present invention. Reference is also made to Fig. 2, which is a schematic pictorial illustration of a balloon catheter 40, in accordance with an embodiment of the present invention.

The position tracking and ablation system 20 is used to determine the position of the balloon catheter 40, seen in an inset 25 of Fig. 1 and in more detail in Fig. 2. The balloon catheter 40 includes a shaft 22 and an inflatable balloon 45 fitted at a distal end of the shaft 22. Typically, the balloon catheter 40 is used for therapeutic treatment, such as spatially ablating cardiac tissue, for example at the left atrium. The catheter 40 is configured to be inserted in a chamber of a heart 26 of a living subject (e.g., a patient 28).

The position tracking and ablation system 20 can determine a position and orientation of the shaft 22 of the balloon catheter 40 based on sensing-electrodes 52 (proximal-electrode 52a and distal-electrode 52b) fitted on the shaft 22, on either side of the inflatable balloon 45 and a magnetic sensor 50 fitted just proximally to proximal-electrode 52a. The proximal-electrode 52a, the distal-electrode 52b, and the magnetic sensor 50 are connected by wires running through the shaft 22 to various driver circuitries in a console 24. In some embodiments, the distal electrode 52b may be omitted. The magnetic sensor 50 may comprise a single axis sensor (SAS), or a double axis sensor (DAS), or a triple axis sensor (TAS), by way of example.

The shaft 22 defines a longitudinal axis 51 (Fig. 2). A center-point 58 (Fig. 2) on the axis 51, which is the origin of the sphere shape of the inflatable balloon 45, defines a nominal position of the inflatable balloon 45. Multiple ablation electrodes 55 are disposed in a circumference over the inflatable balloon 45, which occupy a large area as compared with sensing-electrodes 52a and 52b. Radio frequency power or IRE ablation signals may be supplied to the ablation electrodes 55 to ablate the cardiac tissue.

Typically, the disposed ablation electrodes 55 are evenly distributed along an equator of the inflatable balloon 45, where the equator is generally aligned perpendicular to the longitudinal axis 51 of the distal end of the shaft 22.

The illustration shown in Fig. 2 is chosen purely for the sake of conceptual clarity. Other configurations of sensing-electrodes 52 and ablation electrodes 55 are possible. Additional functionalities may be included in the magnetic sensor 50. Elements which are not relevant to the disclosed embodiments of the invention, such as irrigation ports, are omitted for the sake of clarity.

A physician 30 navigates the balloon catheter 40 to a target location in the heart 26 of the patient 28 by manipulating the shaft 22 using a manipulator 32 near the proximal end of the catheter and/or deflection from a sheath 23. The balloon catheter 40 is inserted, while the inflatable balloon 45 is deflated, through the sheath 23, and only after the balloon catheter 40 is retracted from the sheath 23 is the inflatable balloon 45 inflated and regains its intended functional shape. By containing balloon catheter 40 in a deflated configuration, the sheath 23 also serves to minimize vascular trauma on its way to the target location.

In some embodiments, the manipulator 32 may include a tactile device 73 to provide tactile feedback to the physician 30. In some embodiments, the tactile device 73 may be disposed in a foot pedal operated by the physician 30.

Console 24 comprises a processor 41, typically a general-purpose computer and a suitable front end and interface circuits 44 for generating signals in, and/or receiving signals from, body surface patches 49 which are attached by wires running through a cable 39 and configured to be attached to the chest and to the back of the patient 28. In some embodiments, the body surface patches 49 provide position signals, as described in more detail below. The body surface patches 49 may comprise respective electrodes 75, and/or respective magnetic sensors 77. Each magnetic sensor 77 may comprise a single axis sensor (SAS), or a double axis sensor (DAS), or a triple axis sensor (TAS), by way of example.

Console 24 further comprises a magnetic-sensing sub-system. The patient 28 is placed in a magnetic field generated by a pad containing magnetic field generator coils 42, which are driven by a unit 43 disposed in the console 24. The magnetic field generator coils 42 are configured to generate alternating magnetic fields is a region including the magnetic sensor 50 and the body surface patches 49. The magnetic fields generated by the coils 42 generate direction signals in the magnetic sensor 50 and the magnetic sensors 77, which are then provided as corresponding electrical inputs to the processor 41.

In some embodiments, the processor 41 uses the position-signals received from the sensing-electrodes 52, the magnetic sensor 50 and the ablation electrodes 55 to estimate a position of the balloon catheter 40 inside an organ, such as inside a cardiac chamber. In some embodiments, the processor 41 correlates the position signals received from the electrodes 52, 55 with previously acquired magnetic location-calibrated position signals, to estimate the position of the balloon catheter 40 inside a cardiac chamber. The position coordinates of the sensing-electrodes 52 and the ablation electrodes 55 may be determined by the processor 41 based on, among other inputs, measured impedances, or on proportions of currents distribution, between the electrodes 52, 55 and the body surface patches 49. The console 24 drives a display 27, which shows the distal end of the catheter position inside the heart 26.

The method of position sensing using current distribution measurements and/or external magnetic fields is implemented in various medical applications, for example, in the Carto^{®} system, produced by Biosense Webster Inc. (Irvine, California), and is described in detail in U.S. Patents 5,391,199, 6,690,963, 6,484,118, 6,239,724, 6,618,612, 6,332,089, 7,756,576, 7,869,865, and 7,848,787, in PCT Patent Publication WO 96/05768, and in US Patent Publications 2002/0065455 A1, 2003/0120150 A1 and 2004/0068178 A1.

The Carto^{®}3 system applies an Active Current Location (ACL) impedance-based position-tracking method. In some embodiments, using the above noted ACL method, the processor 41 estimates the positions of the sensing-electrodes 52 and the ablation electrodes 55. In some embodiments, the signals received from the electrodes 52, 55 are correlated with a matrix which maps impedance (or another electrical value) measured by the sensing-electrodes 52, 55 with a position that was previously acquired from magnetic location-calibrated position signals.

In some embodiments, to visualize catheters which do not include a magnetic sensor, the processor 41 may apply an electrical signal-based method, referred to as the Independent Current Location (ICL) method. In the ICL method, the processor 41 calculates a local scaling factor for each voxel of a volume of the balloon catheter 40. The factor is determined using a catheter with multiple electrodes having a known spatial relationship, such as a Lasso-shaped catheter. However, although yielding accurate local scaling (e.g., over several millimeters), ICL may be less accurate when applied to a balloon catheter, whose size is on the order of centimeters. In some embodiments, the processor 41 may apply the disclosed ICL method to scale the balloon catheter shape into a correct one, based on known smaller scale distances between electrodes of a lasso-shaped catheter, as well as based on larger scale distances, themselves based on the known distance between the sensing-electrodes 52 at the ends of the inflatable balloon 45.

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

The medical system 20 may also include an ablation power generator 69 (such as an RF or IRE signal generator) configured to be connected to the catheter 40, and apply an electrical signal between one or more of the electrodes 55 and the proximal electrode 52a or between two or more of the electrodes 55 to ablate tissue in the chamber of the heart.

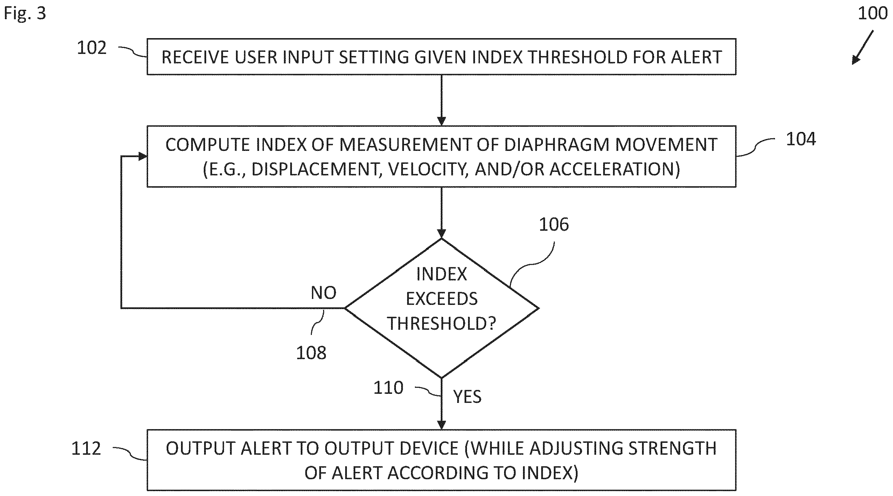

The system 20 may include an audio output device 71 to provide audio messages or alerts, described in more detail with reference to Fig. 3.

The example illustration shown in Fig. 1 is chosen purely for the sake of conceptual clarity. Fig. 1 shows only elements related to the disclosed techniques for the sake of simplicity and clarity. System 20 typically comprises additional modules and elements that are not directly related to the disclosed techniques, and thus are intentionally omitted from Fig. 1 and from the corresponding description.

A balloon catheter is described herein by way of example only. The system 20 may be implemented using any suitable electrode catheter, for example, a lasso catheter, a basket catheter, or a grid catheter.

Reference is now made to Fig. 3, which is a flowchart 100 including a method of operation of the system of Fig. 1. Reference is also made to Fig. 1.

The processor 41 is configured to receive (block 102) a user input (e.g., from the physician 30) setting a threshold related to outputting an alert (and/or performing another action such as controlling the ablation power generator 69 to control, stop, or prevent ablation) when an index of measurement of diaphragm movement exceeds the threshold, described in more detail below.

The processor 41 is configured to compute (block 104) indices of the measurement of diaphragm movement over time (i.e., one index at time 1, another index at time 2, etc.) responsively to one or more position signals provided by the body surface patches 49. When one of the electrodes 55 (Fig. 2) provides an electrical signal (e.g., pacing signal or ablation signal) close to the phrenic nerve (indicated in Fig. 1 using dotted lines 47), the movement of the diaphragm is significantly larger than normal breathing and may therefore be detected using position signals provided by the body surface patches 49. Therefore, the index of the measurement of the diaphragm movement is indicative of proximity of the electrode(s) 55 to the phrenic nerve.

In some embodiments, the processor 41 is configured to compute the indices of the measurement of the diaphragm movement responsively to a measurement of displacement, and/or velocity, and/or acceleration of the diaphragm movement responsively to one or more of the position signals. The velocity and acceleration may be computed based on analyzing the movement of the positions of the body surface patches 49 over time. Alternatively, or additionally, the magnetic sensors 77 may provide signals indicative of acceleration of the body surface patches 49.

In some embodiments, the magnetic sensors 77 of the body surface patches 49 are configured to provide respective position signals responsively to sensing the generated alternating magnetic fields generated by the magnetic field generator coils 42. The processor 41 may be configured to compute the index of the measurement of the diaphragm movement responsively to one or more of the position signals received from the magnetic sensors 77. In some embodiments, the system 20 may include one body surface patch 49 and one corresponding magnetic sensor 77, which provides a position signal used to compute the index of the measurement of the diaphragm movement.

In some embodiments, one or more of the position signals from the magnetic sensors 77 may be processed to compute the position(s) of the corresponding body surface patch(es) 49. The computed position(s) may then be used to compute the index of diaphragm movement. For example, the computed positions may be summed, averaged or otherwise combined to compute the index. The positions may be relative positions, for example, relative to an origin of a magnetic coordinate system, relative to an average position of the chest or diaphragm, or relative to some other given position. In the above computation of the index, the position of the body surface patch 49 closest to the diaphragm on the chest may be used. In some embodiments, the positions of a sub-set of the body surface patches 49 closest to the diaphragm may be used to compute the index. In some embodiments, the computation of the index may weight the computed positions of the (subset of) body surface patches 49 according to the proximity of the corresponding body surface patches 49 to the diaphragm providing a higher weight to patches 49 that are closer to the diaphragm.

In some embodiments, the respective electrodes 75 of the body surface patches 49 are configured to provide respective position signals. The electrodes 75 may detect signals provided by the catheter electrode(s) 52, 55, and/or from other one or ones of the body surface patches 49, and/or from a reference electrode (not shown) placed in the heart 26 or on the back of the patient 28, for example. The processor 41 may be configured to compute the index of the measurement of the diaphragm movement responsively to one or more of the position signals received from the electrodes 75. In some embodiments, the processor 41 may be configured to compute the index of the measurement of the diaphragm movement responsively to one of the position signals received from one of the electrodes.

In some embodiments, the position signals from the electrodes 75 may then be used to compute positions of one or more of the body surface patches 49. One or more of the computed positions may then be used to compute the index as described above with reference to the positions computed for the magnetic sensors 77.

In some embodiments, a distribution of current or impedance values over the body surface patch electrodes 49 may provide an indication of diaphragm movement. Therefore, the index of diaphragm movement may be computed based on the distribution of the current or impedance values of all, or a subset of, the body surface patch electrodes 49.

After the computation of each index, at a decision block 106, the processor 41 is configured to determine if the computed index exceeds the given threshold. If the computed index does not exceed the given threshold (branch 108), the step of block 104 is repeated computing the next index. If the computed index exceeds the given threshold (branch 110), the processor 41 is configured to output (block 112) an alert to an output device 85 (e.g., to the display 27, and/or the audio output device 71, and/or the tactile device 73) responsively to the computed index (e.g., a strength and/or frequency of the alert may be dependent on a value of the index, as described in more detail below). Therefore, the processor 41 is configured to output the alert to the output device 85 (e.g., to the display 27, and/or the audio output device 71, and/or the tactile device 73) responsively to the computed index exceeding the given threshold. The alert may comprise any one or more of the following: a current value 81 of the index; an alert message 83 displayed on the display 27; a tactile alert provided to the physician via the tactile device 73, e.g., via the manipulator 32 held by the physician 30, and/or via a foot peddle (not shown) operated by the physician 30; an audio alert via the audio output device 71 (e.g., an alarm and/or a verbal warning); and a graph showing a value of the index over time.

In some embodiments, the processor 41 is configured to output the alert to the output device (e.g., to the display 27, and/or the audio output device 71, and/or the tactile device 73) while adjusting a strength and/or frequency of the alert responsively to the computed indices over time. For example, the alarm may be made louder and/or with a higher frequency, and/or the alert message may be displayed in a stronger color and/or brighter and/or bigger font, and/or the tactile alert may be made to be stronger and/or have a higher frequency, according to the value of the indices, so that as the catheter electrode(s) 55 get closer to the phrenic nerve the strength and/or frequency of the alert increases.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and sub-combinations of the various features described hereinabove, as well as variations and modifications thereof which would occur to persons skilled in the art upon reading the foregoing description and which are not disclosed in the prior art.

### Aspects of the invention:

1. An ablation method, comprising:
   applying an electrical signal to at least one electrode of a catheter inserted into a chamber of a heart of a living subject to ablate tissue of the chamber;
   providing at least one position signal by at least one body surface patch attached to a body surface of the living subject;
   computing an index of a measurement of diaphragm movement responsively to the at least one position signal; and
   performing an action responsively to the computed index.
2. The method according to aspect 1, further comprising controlling an ablation power generator responsively to the computed index.
3. The method according to aspect 1, further comprising outputting an alert to an output device responsively to the computed index.
4. The method according to aspect 3, wherein the outputting includes outputting the alert to the output device responsively to the computed index exceeding a given threshold.
5. The method according to aspect 4, further comprising receiving a user input setting the given threshold.
6. The method according to aspect 3, wherein the alert is selected from a group consisting of: a current value of the index; an alert message; a tactile alert; and an audio alert.
7. The method according to aspect 3, further comprising computing indices of the measurement of diaphragm movement over time responsively to at least one of the position signals, wherein the outputting includes outputting the alert to the output device while adjusting a strength of the alert responsively to the computed indices over time.
8. The method according to aspect 1, wherein the index of the measurement of the diaphragm movement is indicative of proximity of the at least one electrode to the phrenic nerve.
9. The method according to aspect 1, further comprising generating alternating magnetic fields in a region including the at least one body surface patch, wherein:
   the providing includes at least one magnetic sensor of the at least one body surface patch providing the at least one position signal responsively to sensing the generated alternating magnetic fields; and
   the computing includes computing the index of the measurement of the diaphragm movement responsively to the at least one position signal received from the at least magnetic sensor.
10. The method according to aspect 1, wherein:
   the providing includes at least one respective electrode of the at least one body surface patch providing the at least one position signal; and
   the computing includes computing the index of the measurement of the diaphragm movement responsively to the at least one position signal received from the at least one respective electrode.
11. The method according to aspect 1, wherein the computing includes computing the index of the measurement of the diaphragm movement responsively to a measurement of velocity of the diaphragm movement.
12. The method according to aspect 1, wherein the computing includes computing the index of the measurement of the diaphragm movement responsively to a measurement of acceleration of the diaphragm movement.

## Claims

1. An ablation system, comprising:
a catheter including at least one electrode, and configured to be inserted into a chamber of a heart of a living subject;
an ablation power generator configured to apply an electrical signal to the at least one electrode to ablate tissue of the chamber;
at least one body surface patch configured to be applied to a body surface of the living subject, and provide at least one position signal; and
a processor configured to:
compute an index of a measurement of diaphragm movement responsively to the at least one position signal; and
perform an action responsively to the computed index.

2. The system according to claim 1, wherein the processing is configured to control the ablation power generator responsively to the computed index.

3. The system according to claim 1, wherein the processing is configured to output an alert to an output device responsively to the computed index.

4. The system according to claim 3, wherein the processor is configured to output the alert to the output device responsively to the computed index exceeding a given threshold.

5. The system according to claim 4, wherein the processor is configured to receive a user input setting the given threshold.

6. The system according to claim 3, wherein the alert is selected from a group consisting of: a current value of the index; an alert message; a tactile alert; and an audio alert.

7. The system according to claim 3, wherein the processor is configured to:
compute indices of the measurement of diaphragm movement over time responsively to at least one of the position signals; and
output the alert to the output device while adjusting a strength of the alert responsively to the computed indices over time.

8. The system according to claim 1, wherein the index of the measurement of the diaphragm movement is indicative of proximity of the at least one electrode to the phrenic nerve.

9. The system according to claim 1, further comprising magnetic field generator coils configured to generate alternating magnetic fields in a region including the at least one body surface patch, wherein:
the at least one body surface patch comprises at least one magnetic sensor configured to provide the at least one position signal responsively to sensing the generated alternating magnetic fields; and
the processor is configured to compute the index of the measurement of the diaphragm movement responsively to the at least one position signal received from the at least magnetic sensor.

10. The system according to claim 1, wherein:
the at least one body surface patch comprises at least one respective electrode configured to provide the at least one position signal; and
the processor is configured to compute the index of the measurement of the diaphragm movement responsively to the at least one position signal received from the at least one respective electrode.

11. The system according to claim 1, wherein the processor is configured to compute the index of the measurement of the diaphragm movement responsively to a measurement of velocity of the diaphragm movement.

12. The system according to claim 1, wherein the processor is configured to compute the index of the measurement of the diaphragm movement responsively to a measurement of acceleration of the diaphragm movement.
